# EUROPÄISCHE PATENTANMELDUNG

(11) **EP 3 570 289 A1**
(43) Veröffentlichungstag der Anmeldung: **20.11.2019**
(21) Anmeldenummer: 18173169.6
(22) Anmeldetag: 18.05.2018
(51) Int. Cl.: G16H 40/63, G01R 33/28

(54) **VERFAHREN ZU EINER UNTERSTÜTZUNG EINER POSITIONIERUNG EINER ZUBEHÖREINHEIT AN EINEM PATIENTEN FÜR EINE MAGNETRESONANZUNTERSUCHUNG**

(71) Anmelder: Siemens Healthcare GmbH, 91052 Erlangen (DE)
(72) Erfinder: Hesels, Katharina, 91058 Erlangen (DE); Ferguson, George William, 91056 Erlangen (DE); Norosinski, Sabine, 91080 Spardorf (DE); Vollmer, Jonas, 91054 Erlangen (DE); Achleitner, Roman, 90419 Nürnberg (DE)

(57) **Zusammenfassung**

Die Erfindung betrifft ein Verfahren zu einer Unterstützung einer Positionierung einer Zubehöreinheit an einem Patienten für eine Magnetresonanzuntersuchung, mit den folgenden Schritten:
- Erfassen von Positionsdaten des Patienten mittels einer Erfassungseinheit,
- Berechnen einer individuellen Sollposition der Zubehöreinheit mittels einer Berechnungseinheit, wobei das Berechnen der individuellen Sollposition der Zubehöreinheit anhand der Positionsdaten und anhand einer Untersuchungsinformation und/oder anhand von Daten von Zubehöreinheiten erfolgt, und
- Projizieren der individuellen Sollposition der Zubehöreinheit auf den Patienten mittels einer Projektionseinheit, wobei die Zubehöreinheit ein charakteristisches Muster umfasst, das an der individuellen Sollposition projiziert wird.

## Beschreibung

Die vorliegende Erfindung betrifft ein Verfahren zu einer Unterstützung einer Positionierung einer Zubehöreinheit an einem Patienten für eine Magnetresonanzuntersuchung. Des Weiteren betrifft die vorliegende Erfindung eine Magnetresonanzvorrichtung, die zur Ausführung eines Verfahrens zu einer Unterstützung einer Positionierung einer Zubehöreinheit an einem Patienten für eine Magnetresonanzuntersuchung ausgelegt ist, sowie ein entsprechendes Computerprogrammprodukt, das zur Ausführung eines Verfahrens zu einer Unterstützung einer Positionierung einer Zubehöreinheit an einem Patienten für eine Magnetresonanzuntersuchung ausgelegt ist, und ein elektronisch lesbarer Datenträger mit dem Computerprogramm.

Ein wesentlicher Faktor für eine Bildqualität bei Magnetresonanzuntersuchungen ist eine ideale und/oder optimale Positionierung von möglichen Zubehöreinheiten, wie beispielsweise lokale Hochfrequenzantenneneinheiten, am Patienten, insbesondere an einem zu untersuchenden Bereich des Patienten. Die Positionierung der Zubehöreinheiten wird von einem medizinischen Bedienpersonal durchgeführt, so dass die optimale Positionierung der Zubehöreinheiten oft von einem Erfahrungswert des medizinischen Bedienpersonals abhängig ist. Wird die Positionierung der Zubehöreinheiten von einem unerfahrenen und/oder ungeübten medizinischen Bedienpersonal durchgeführt, kann dies zu Problemen bei der Positionierung führen und damit auch zu Problemen bei der Bildqualität führen.

Verfahren zur Positionierung sind aus DE 10 2016 209 297 A1 sowie die DE 10 2016 208 018 A1 bekannt.

Der vorliegenden Erfindung liegt insbesondere die Aufgabe zugrunde, eine Vorbereitung eines Patienten für eine Magnetresonanzuntersuchung für ein medizinisches Bedienpersonal zu vereinfachen. Die Aufgabe wird durch die Merkmale der unabhängigen Ansprüche gelöst. Vorteilhafte Ausgestaltungen sind in den Unteransprüchen beschrieben.

Die Erfindung geht aus von einem Verfahren zu einer Unterstützung einer Zubehöreinheit an einem Patienten für eine Magnetresonanzuntersuchung, mit den folgenden Schritten:
- Erfassen von Positionsdaten des Patienten mittels einer Erfassungseinheit,
- Berechnen einer individuellen Sollposition der Zubehöreinheit mittels einer Berechnungseinheit, wobei das Berechnen der individuellen Sollposition der Zubehöreinheit anhand der Positionsdaten und anhand einer Untersuchungsinformation und/oder anhand der Daten von Zubehöreinheiten erfolgt, und
- Projizieren der individuellen Sollposition der Zubehöreinheit auf den Patienten mittels einer Projektionseinheit, wobei die Zubehöreinheit ein charakteristisches Muster umfasst, das an der individuellen Sollposition projiziert wird.

Vor einem Start einer Magnetresonanzuntersuchung muss zunächst der zu untersuchende Patient von einem medizinischen Bedienpersonal für diese Magnetresonanzuntersuchung vorbereitet werden. Zu Vorbereitung des Patienten zählt beispielsweise ein Positionieren des Patienten auf einer Patientenlagerungsvorrichtung. Die Positionierung des Patienten auf der Patientenlagerungsvorrichtung ist zudem abhängig von einem zu untersuchenden Bereich des Patienten. Beispielsweise muss der Patient für eine Kopfuntersuchung derart auf der Patientenlagerungsvorrichtung platziert werden, dass der Patient mit dem Kopf voran in einen Patientenaufnahmebereich der Magnetresonanzvorrichtung eingefahren wird. Für eine Fußuntersuchung und/oder eine Knieuntersuchung dagegen muss der Patient derart auf der Patientenlagerungsvorrichtung platziert werden, dass der Patient mit den Füssen voran in einen Patientenaufnahmebereich der Magnetresonanzvorrichtung eingefahren wird.

Neben einer Positionierung des Patienten werden zur Vorbereitung des Patienten für die Magnetresonanzuntersuchung auch noch, sofern erforderlich, Zubehöreinheiten an dem Patienten angebracht. Derartige Zubehöreinheiten können beispielsweise eine lokale Hochfrequenzantenneneinheit für eine Magnetresonanzuntersuchung am Patienten sein. Zudem kann die Zubehöreinheit auch eine EKG-Einheit und/oder Atemsensoren und/oder Positionierungshilfen, wie beispielsweise ein Kissen oder ein vorgeformter Schaum, und/oder weitere, dem Fachmann als sinnvoll erscheinende Zubehöreinheiten umfassen. Diese Zubehöreinheiten müssen von dem medizinischen Bedienpersonal korrekt, insbesondere mit der korrekten Orientierung an der korrekten Position, am Patienten positioniert werden.

Das Erfassen der Positionsdaten des Patienten erfolgt vorzugsweise selbsttätig und/oder automatisch mittels der Erfassungseinheit. Die Erfassungseinheit kann dabei an einer Scannereinheit der Magnetresonanzvorrichtung angeordnet sein oder auch an einer Wand oder Zimmerdecke eines Untersuchungsraums, innerhalb dessen die Scannereinheit der Magnetresonanzvorrichtung angeordnet ist. Vorzugsweise weist die Erfassungseinheit einen Erfassungsbereich auf, wobei der Erfassungsbereich einen Bereich umfasst, in dem der Patient für die Magnetresonanzuntersuchung vorbereitet wird. Dieser Erfassungsbereich umfasst bevorzugt einen Bereich vor der Scannereinheit der Magnetresonanzvorrichtung, wobei dieser Bereich an eine Frontseite der Scannereinheit angrenzt.

Die Positionsdaten des Patienten umfassen bevorzugt eine Lage und/oder Orientierung und/oder eine Ausdehnung, insbesondere eine Größe und/oder eine Länge und/oder eine Breite und/oder eine Höhe, des Patienten. Alternativ oder zusätzlich können die Positionsdaten des Patienten auch eine Position des Patienten auf der Patientenlagerungsvorrichtung und/oder bezüglich der Patientenlagerungsvorrichtung umfassen. Zusätzlich können die Positionsdaten des Patienten auch nur einzelne Körperbereiche und/oder Körperregionen des Patienten, vorzugsweise den zu untersuchenden Körperbereich des Patienten, umfassen, wie beispielsweise Abmessungen eines Arms oder des Kopfs oder des Oberkörpers usw. umfassen.

Die erfassten Positionsdaten des Patienten werden bevorzugt mittels einer Datenübertragungseinheit von der Erfassungseinheit an die Berechnungseinheit übertragen. Hierbei kann es vorgesehen sein, dass die Berechnungseinheit die erfassten Positionsdaten des Patienten auch aktiv von der Erfassungseinheit abruft. Die Datenübertragung zwischen der Berechnungseinheit und der Erfassungseinheit kann dabei kabelgebunden oder auch kabellos oder auch drahtlos erfolgen.

Das Berechnen der individuellen Sollposition der Zubehöreinheit erfolgt mittels der Berechnungseinheit. Vorzugsweise erfolgt das Berechnen der individuellen Sollposition der Zubehöreinheit automatisch und/oder selbsttätig mittels der Berechnungseinheit. Die Berechnungseinheit weist hierzu einen Prozessor auf. Des Weiteren umfasst die Berechnungseinheit auch entsprechende Berechnungsprogramme und/oder eine Berechnungssoftware, die bei einem Ausführen ein Verfahren zu einem Unterstützen einer Vorbereitung eines Patienten für eine Magnetresonanzuntersuchung ausführen. Die Berechnungsprogramme und/oder Berechnungssoftware können dabei innerhalb einer Speichereinheit der Berechnungseinheit und/oder der Magnetresonanzvorrichtung gespeichert sein. Zudem können die Berechnungsprogramme und/oder Berechnungssoftware auch in einem externen Speicher, wie beispielsweise einer Cloud, hinterlegt sein, wobei die Berechnungseinheit hierbei mittels eines Datennetzwerks auf die Berechnungsprogramme und/oder Berechnungssoftware zugreifen kann. Die Berechnungseinheit kann innerhalb einer Steuerungseinheit der Magnetresonanzvorrichtung integriert sein. Alternativ oder zusätzlich kann die Berechnungseinheit auch als selbstständige Einheit ausgebildet sein.

Die Untersuchungsinformation umfasst beispielsweise eine Untersuchungsregion und/oder einen zu untersuchenden Bereich des Patienten. Die Untersuchungsregion kann bereits in einer Patientendatenbank hinterlegt sein, auf die die Berechnungseinheit zugreifen kann. Beispielsweise kann die Untersuchungsregion bereits bei einer Patientenregistrierung erfasst werden und in der Patientendatenbank hinterlegt werden. Zudem ist es auch denkbar, dass die Untersuchungsregion während der Vorbereitung des Patienten für die Magnetresonanzuntersuchung von dem medizinischen Bedienpersonal übermittelt wird, wie beispielsweise mittels einer manuellen Eingabe der Untersuchungsregion. Die Untersuchungsinformation kann beispielsweise auch ein Krankheitsbild des Patienten umfassen, anhand dessen eine Untersuchungsregion festgelegt wird. Das Festlegen der Untersuchungsregion anhand eines Krankheitsbilds kann auch automatisch und/oder selbsttätig mittels der Berechnungseinheit erfolgen. Beispielsweise kann bei einem Krankheitsbild "Kopfschmerzen des Patienten" anhand dessen die Untersuchungsregion zunächst auf den Kopf des Patienten festgelegt werden kann.

Die Daten der Zubehöreinheiten sind bevorzugt innerhalb einer Datenbank hinterlegt, wobei die Berechnungseinheit mittels des Datennetzwerkes auf die Daten der Zubehöreinheiten der Datenbank zugreifen kann. Die Daten der Zubehöreinheiten umfassen typischerweise eine vordefinierte Position der einzelnen Zubehöreinheiten bezüglich der Patientenlagerungsvorrichtung und/oder bezüglich eines Durchschnittspatienten. Alternativ oder zusätzlich können die Daten der Zubehöreinheiten auch eine Geometrie, insbesondere eine Länge und/oder eine Breite und/oder eine Höhe, der einzelnen Zubehöreinheiten umfassen.

Die individuelle Sollposition der Zubehöreinheit umfasst bevorzugt eine Sollposition der Zubehöreinheit an dem Patienten und/oder auf dem Patienten. Die individuelle Sollposition der Zubehöreinheit umfasst somit eine Position der Zubehöreinheit, die die Zubehöreinheit während der Magnetresonanzuntersuchung bezüglich des Patienten einnehmen soll. Die individuelle Sollposition kann dabei eine patientenspezifischen Sollposition umfassen, bei die gewünschte Position der Zubehöreinheit in Abhängigkeit von den Positionsdaten des Patienten, insbesondere in Abhängigkeit einer Anatomie des Patienten, ermittelt wird.

Unter einem charakteristischen Muster der Zubehöreinheit soll insbesondere ein Muster auf der Oberfläche der Zubehöreinheit verstanden werden. Vorzugsweise kann anhand des Musters auf der Oberfläche der Zubehöreinheit eine Identifizierung der Zubehöreinheit erfolgen. Das charakteristische Muster kann dabei ein unregelmäßiges Muster umfassen, so dass anhand eines Musterausschnitts eine exakte Position und/oder Orientierung der Zubehöreinheit ermittelt werden kann.

Die berechnete individuelle Sollposition der Zubehöreinheit wird bevorzugt mittels einer Datenübertragungseinheit von der Berechnungseinheit an die Projektionseinheit übertragen. Hierbei kann es auch vorgesehen sein, dass die Berechnungseinheit die berechnete individuelle Sollposition der Zubehöreinheit aktiv an die Projektionseinheit übermittelt und/oder sendet. Die Datenübertragung zwischen der Berechnungseinheit und der Projektionseinheit kann dabei kabelgebunden oder auch kabellos oder auch drahtlos erfolgen.

Das Projizieren der individuellen Sollposition der Zubehöreinheit erfolgt bevorzugt mittels der Projektionseinheit. Die Projektionseinheit kann dabei an der Scannereinheit der Magnetresonanzvorrichtung angeordnet sein oder auch an einer Wand oder Zimmerdecke des Untersuchungsraums, innerhalb dessen die Scannereinheit der Magnetresonanzvorrichtung angeordnet ist. Vorzugsweise ist ein Projektionsbereich der Projektionseinheit auf den Patienten und/oder die Patientenlagerungsvorrichtung während einer Vorbereitung des Patienten für die Magnetresonanzuntersuchung gerichtet. Dieser Projektionsbereich umfasst bevorzugt einen Bereich vor der Scannereinheit der Magnetresonanzvorrichtung, wobei dieser Bereich an eine Frontseite der Scannereinheit angrenzt. Das Projizieren der Positionsinformation des Objekts erfolgt bevorzugt automatisch und/oder selbsttätig mittels der Projektionseinheit.

Die Erfindung ermöglicht eine vorteilhafte Unterstützung eines medizinischen Bedienpersonals während einer Vorbereitung eines Patienten für eine medizinische Bildgebungsuntersuchung. Insbesondere kann derart für ein unerfahrenes und/oder ungeübtes medizinisches Bedienpersonal eine einfache und zeitsparende Hilfestellung zur Positionierung des Patienten und/oder zur Anordnung und/oder Positionierung von Zubehöreinheiten bereitgestellt werden. Folglich kann durch die Unterstützung für eine optimierte Lagerung des Patienten und/oder der Zubehöreinheit eine Fehlpositionierung verhindert werden und damit eine Bildqualität vorteilhaft erhöht werden. Hierdurch können auch Messwiederholungen aufgrund von Fehlpositionierungen vorteilhaft verhindert werden.

Zudem kann eine eindeutige Identifizierung einer Zubehöreinheit erfolgen. Zum einen kann mittels der Erfassungseinheit die Zubehöreinheit anhand des erfassten charakteristischen Musters identifiziert werden und damit dem Benutzer vorteilhaft ein Positionierungsvorschlag angezeigt werden. Zum anderen kann dem Benutzer für die geplante Untersuchung ein Vorschlag für eine Auswahl einer Zubehöreinheit unterbreitet werden, wobei der Benutzer anhand des charakteristischen Musters die Zubehöreinheit auswählen kann und eine Verwechslung der Zubehöreinheit somit verhindert werden kann.

In einer vorteilhaften Weiterbildung der Erfindung kann es vorgesehen sein, dass die Erfassungseinheit eine Kamera umfasst. Die Erfassungseinheit umfasst bevorzugt eine 2D-Kamera und/oder eine 3D-Kamera. Hierdurch kann eine besonders kostengünstige Erfassungseinheit zur Erfassung der Positionsdaten des Patienten bereitgestellt werden.

Alternativ oder zusätzlich kann die Erfassungseinheit auch eine Sensoreinheit umfassen, welche Sensoreinheit die Positionsdaten des Patienten erfasst. Dabei kann die Sensoreinheit innerhalb der Patientenlagerungsvorrichtung integriert sein, wie beispielsweise Drucksensoren und/oder Hochfrequenzsensoren umfassen, welche Sensoren innerhalb einer Liegefläche der Patientenlagerungsvorrichtung integriert sind, so dass anhand einer Druckverteilung auf eine Lage und/oder Orientierung und/oder Größe Und/oder Position des Patienten geschlossen werden kann.

In einer vorteilhaften Weiterbildung der Erfindung kann es vorgesehen sein, dass die Zubehöreinheit eine lokale Hochfrequenzantenneneinheit umfasst. Dies ermöglich eine einfache und schnelle Positionierung einer lokalen Hochfrequenzantenneneinheit auch für ein ungeübtes und/oder unerfahrenes Bedienpersonal.

In einer vorteilhaften Weiterbildung der Erfindung kann es vorgesehen sein, dass die Positionsdaten des Patienten eine Kontur des Patienten umfassen. Dabei soll unter einer Kontur des Patienten insbesondere ein Umriss des Körpers des Patienten verstanden werden, wobei die Kontur und/oder der Umriss des Patienten anhand eines Patientenmodells bestimmt und/oder berechnet werden kann. Das Patientenmodell kann beispielsweise ein 2D-Patientenmodell oder auch ein 3D-Patientenmodell umfassen. Demzufolge kann die Kontur des Patienten eine 2D-Kontur oder auch eine 3D-Kontur des Patienten umfassen. Hierdurch kann eine besonders exakte Bestimmung und/oder Berechnung der patientenspezifischen und/oder der individuellen Sollposition der Zubehöreinheit ermöglicht werden. Insbesondere können hierbei bei der Bestimmung der individuellen Sollposition der Zubehöreinheit vorteilhaft anatomische Besonderheiten des Patienten berücksichtigt werden.

In einer vorteilhaften Weiterbildung der Erfindung kann es vorgesehen sein, dass die Berechnungseinheit für die Berechnung der individuellen Sollposition der Zubehöreinheit einen Algorithmus aufweist, der anhand der Positionsdaten des Patienten eine patientenspezifische Sollposition der Zubehöreinheit bestimmt. Unter einer patientenspezifischen Sollposition soll hierbei insbesondere eine Sollposition der Zubehöreinheit verstanden werden, die speziell an eine Figur und/oder eine Anatomie des Patienten angepasst ist. Mittels des Algorithmus kann somit besonders schnell und zeitsparend die individuelle Sollposition der Zubehöreinheit an den Patienten angepasst werden.

In einer vorteilhaften Weiterbildung der Erfindung kann es vorgesehen sein, dass sich die einzelnen Zubehöreinheiten anhand ihres charakteristischen Musters unterscheiden, wodurch eine einfache Identifizierung der entsprechenden Zubehöreinheit anhand ihres charakteristischen Musters erfolgen kann. Insbesondere kann derart auch eine Auswahl der Zubehöreinheit für ein ungeübtes und/oder unerfahrenes medizinisches Bedienpersonal vereinfacht werden.

In einer vorteilhaften Weiterbildung der Erfindung kann es vorgesehen sein, dass eine Bestimmung der Zubehöreinheit anhand des charakteristischen Musters erfolgt. Die Zubehöreinheit kann dabei von der Berechnungseinheit anhand der Untersuchungsregion oder weiteren Patientendaten ausgewählt werden und dem Benutzer das charakteristische Muster mitgeteilt werden. Der Benutzer kann dann anhand des charakteristischen Musters die korrekte Zubehöreinheit aus der Vielzahl an Zubehöreinheiten auswählen und/oder bestimmen. Alternativ oder Zusätzlich kann die Zubehöreinheit auch vom Benutzer, insbesondere dem medizinischen Bedienpersonal, ausgewählt werden, wobei anhand des von der Erfassungseinheit erfassten charakteristischen Musters der Zubehöreinheit die Zubehöreinheit bestimmt und/oder identifiziert werden kann und damit auch eine Position, insbesondere die individuelle Sollposition, der Zubehöreinheit von der Berechnungseinheit festgelegt werden kann.

In einer vorteilhaften Weiterbildung der Erfindung kann es vorgesehen sein, dass die Berechnungseinheit für eine Berechnung der individuellen Sollposition der Zubehöreinheit auf eine Datenbank zurückgreift, in der alle zur Verfügung stehenden Zubehöreinheiten mit charakteristischen Muster und/oder mit vordefinierter Position für einen Durchschnittspatienten gespeichert sind. Hierdurch kann vorteilhaft eine automatische Auswahl und/oder Berechnung der individuellen Sollposition der Zubehöreinheit für alle zur Verfügung stehenden Zubehöreinheiten erfolgen. Unter allen zur Verfügung stehenden Zubehöreinheiten sollen hierbei insbesondere alle Zubehöreinheiten verstanden werden, die vor Ort, also am Ort der Magnetresonanzvorrichtung, für eine Magnetresonanzuntersuchung zur Verfügung stehen und/oder vorhanden sind.

In einer vorteilhaften Weiterbildung der Erfindung kann es vorgesehen sein, dass eine Positionierung der Zubehöreinheit bei einer Übereinstimmung des projizierten charakteristischen Musters mit dem charakteristischen Muster auf der Zubehöreinheit erfolgt. Hierdurch kann auch für ein unerfahrenes und/oder ein ungeübtes medizinisches Bedienpersonal eine einfache Positionierung und/oder Anordnung der Zubehöreinheit am Patienten erfolgen.

Des Weiteren geht die Erfindung aus von einer Magnetresonanzvorrichtung, die zu einer Ausführung eines Verfahrens zu einer Unterstützung einer Positionierung einer Zubehöreinheit an einem Patienten für eine Magnetresonanzuntersuchung ausgelegt ist, wobei die Magnetresonanzvorrichtung eine Scannereinheit, eine Patientenlagerungsvorrichtung zur Lagerung des Patienten für die Magnetresonanzuntersuchung, eine Erfassungseinheit zur Erfassung von Positionsdaten des Patienten, eine Berechnungseinheit zur Berechnung einer individuellen Sollposition einer Zubehöreinheit und eine Projektionseinheit zur Projektion der individuellen Sollposition der Zubehöreinheit aufweist.

Die Erfindung ermöglicht eine vorteilhafte Unterstützung eines medizinischen Bedienpersonals während einer Vorbereitung eines Patienten für eine medizinische Bildgebungsuntersuchung. Insbesondere kann derart für ein unerfahrenes und/oder ungeübtes medizinisches Bedienpersonal eine einfache und zeitsparende Hilfestellung zur Positionierung des Patienten und/oder zur Anordnung und/oder Positionierung von Zubehöreinheiten bereitgestellt werden. Folglich kann durch die Unterstützung für eine optimierte Lagerung des Patienten und/oder der Zubehöreinheit eine Fehlpositionierung verhindert werden und damit eine Bildqualität vorteilhaft erhöht werden. Hierdurch können auch Messwiederholungen aufgrund von Fehlpositionierungen vorteilhaft verhindert werden.

Zudem kann eine eindeutige Identifizierung einer Zubehöreinheit erfolgen. Zum einen kann mittels der Erfassungseinheit die Zubehöreinheit anhand des erfassten charakteristischen Musters identifiziert werden und damit dem Benutzer vorteilhaft ein Positionierungsvorschlag angezeigt werden. Zum anderen kann dem Benutzer für die geplante Untersuchung ein Vorschlag für eine Auswahl einer Zubehöreinheit unterbreitet werden, wobei der Benutzer anhand des charakteristischen Musters die Zubehöreinheit auswählen kann und eine Verwechslung der Zubehöreinheit somit verhindert werden kann.

Die Vorteile der erfindungsgemäßen Magnetresonanzvorrichtung entsprechen im Wesentlichen den Vorteilen des erfindungsgemäßen Verfahrens zu einer Unterstützung einer Positionierung einer Zubehöreinheit an einem Patienten für eine Magnetresonanzuntersuchung, welche Vorteile vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

Des Weiteren geht die Erfindung aus von einem Computerprogrammprodukt, welches ein Programm umfasst und direkt in einem Speicher einer programmierbaren Systemsteuereinheit einer Magnetresonanzvorrichtung ladbar ist, mit Programmmitteln, um ein Verfahren zu einer Unterstützung einer Positionierung einer Zubehöreinheit an einem Patienten für eine Magnetresonanzuntersuchung auszuführen, wenn das Programm in der Systemsteuereinheit der Magnetresonanzvorrichtung ausgeführt wird. Dabei benötigt das Computerprogramm eventuell Programmmittel, z.B. Bibliotheken und Hilfsfunktionen, um die entsprechenden Ausführungsformen des Verfahrens zu realisieren. Das Computerprogramm kann dabei eine Software mit einen Quellcode, der noch compiliert und gebunden oder der nur interpretiert werden muss, oder einen ausführbaren Softwarecode umfassen, der zur Ausführung nur noch in eine entsprechende Recheneinheit zu laden ist.

Des Weiteren geht die Erfindung aus von einem computerlesbaren Datenträger, welcher ein Programm umfasst, das zu einer Ausführung eines Verfahrens zu einer Unterstützung einer Positionierung einer Zubehöreinheit an einem Patienten für eine Magnetresonanzuntersuchung vorgesehen ist.

Weitere Vorteile, Merkmale und Einzelheiten der Erfindung ergeben sich aus dem im Folgenden beschriebenen Ausführungsbeispiel sowie anhand der Zeichnungen.

Es zeigen:
- Fig. 1: eine erfindungsgemäße Magnetresonanzvorrichtung in einer schematischen Darstellung,
- Fig. 2: ein Verfahren zu einer Unterstützung einer Positionierung einer Zubehöreinheit an einem Patienten für eine Magnetresonanzuntersuchung und
- Fig. 3: eine Darstellung einer Projektion eines charakteristischen Musters der Zubehöreinheit an der individuellen Sollposition der Zubehöreinheit.

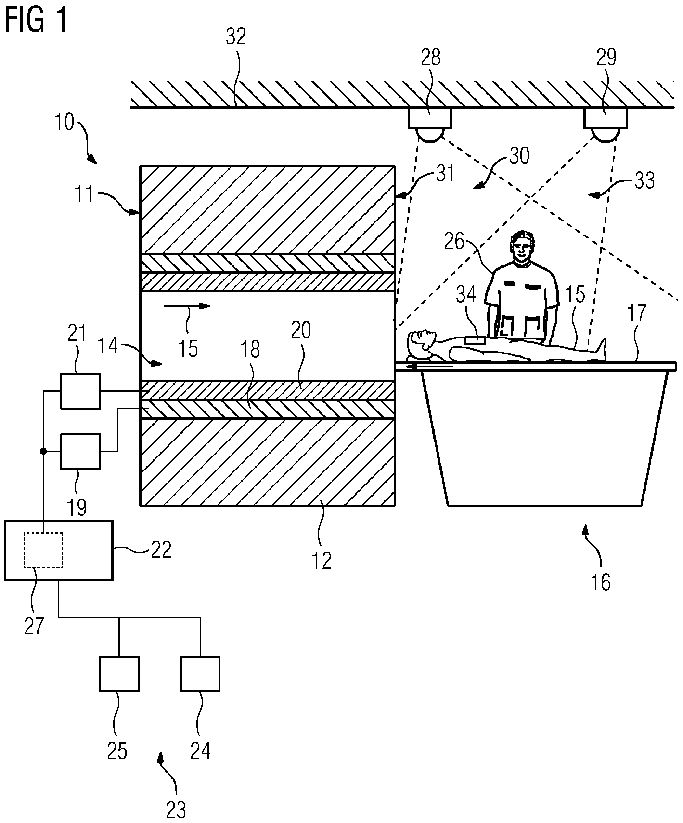

In Fig. 1 ist eine Magnetresonanzvorrichtung 10 dargestellt, die eine Scannereinheit 11, die von einer Magneteinheit gebildet ist, mit einen supraleitenden Hauptmagneten 12 umfasst zu einem Erzeugen eines starken und insbesondere konstanten Hauptmagnetfelds 13. Zudem weist die Magnetresonanzvorrichtung 10 einen Patientenaufnahmebereich 14 auf zu einer Aufnahme eines Patienten 15. Der Patientenaufnahmebereich 14 im vorliegenden Ausführungsbeispiel ist zylinderförmig ausgebildet und in einer Umfangsrichtung von der Scannereinheit 11 zylinderförmig umgeben. Grundsätzlich ist jedoch eine davon abweichende Ausbildung des Patientenaufnahmebereichs 14 jederzeit denkbar. Der Patient 15 kann mittels einer Patientenlagerungsvorrichtung 16 der Magnetresonanzvorrichtung 10 in den Patientenaufnahmebereich 14 geschoben und/oder gefahren werden. Die Patientenlagerungsvorrichtung 16 weist hierzu einen innerhalb des Patientenaufnahmebereichs 14 bewegbar ausgestalteten Patiententisch 17 auf.

Die Scannereinheit 11 weist weiterhin eine Gradientenspuleneinheit 18 zu einer Erzeugung von Magnetfeldgradienten auf, die für eine Ortskodierung während einer Bildgebung verwendet werden. Die Gradientenspuleneinheit 18 wird mittels einer Gradientensteuereinheit 19 der Magnetresonanzvorrichtung 10 gesteuert. Die Scannereinheit 11 umfasst weiterhin eine Hochfrequenzantenneneinheit 20 zu einer Anregung einer Polarisation, die sich in dem von dem Hauptmagneten 12 erzeugten Hauptmagnetfeld 13 einstellt. Die Hochfrequenzantenneneinheit 20 wird von einer Hochfrequenzantennensteuereinheit 21 der Magnetresonanzvorrichtung 10 gesteuert und strahlt hochfrequente Magnetresonanzsequenzen in einen Untersuchungsraum, der im Wesentlichen von einem Patientenaufnahmebereich 14 der Magnetresonanzvorrichtung 10 gebildet ist, ein.

Zu einer Steuerung des Hauptmagneten 12, der Gradientensteuereinheit 19 und zur Steuerung der Hochfrequenzantennensteuereinheit 21 weist die Magnetresonanzvorrichtung 10 eine Systemsteuereinheit 22 auf. Die Systemsteuereinheit 22 steuert zentral die Magnetresonanzvorrichtung 10, wie beispielsweise das Durchführen einer vorbestimmten bildgebenden Gradientenechosequenz. Zudem umfasst die Systemsteuereinheit 22 eine nicht näher dargestellte Auswerteeinheit zu einer Auswertung von medizinischen Bilddaten, die während der Magnetresonanzuntersuchung erfasst werden.

Des Weiteren umfasst die Magnetresonanzvorrichtung 10 eine Benutzerschnittstelle 23, die mit der Systemsteuereinheit 22 verbunden ist. Steuerinformationen wie beispielsweise Bildgebungsparameter, sowie rekonstruierte Magnetresonanzbilder können auf einer Anzeigeeinheit 24, beispielsweise auf zumindest einem Monitor, der Benutzerschnittstelle 23 für ein medizinisches Bedienpersonal 26 angezeigt werden. Weiterhin weist die Benutzerschnittstelle 23 eine Eingabeeinheit 25 auf, mittels der Informationen und/oder Parameter während eines Messvorgangs von dem medizinischen Bedienpersonal 26 eingegeben werden können.

Des Weiteren umfasst die Magnetresonanzvorrichtung 10 eine Berechnungseinheit 27, eine Erfassungseinheit 28 und eine Projektionseinheit 29. Mittels der Berechnungseinheit 27, der Erfassungseinheit 28 und der Projektionseinheit 29 kann ein Verfahren zu einer Unterstützung einer Vorbereitung eines Patienten 15 für eine Magnetresonanzuntersuchung ausgeführt werden. Das Verfahren zur Unterstützung einer Vorbereitung eines Patienten 15 für eine Magnetresonanzuntersuchung wird dabei automatisch und/oder selbsttätig mittels der Magnetresonanzvorrichtung 10, insbesondere der Berechnungseinheit 27 zusammen mit der Erfassungseinheit 28 und der Projektionseinheit 29 ausgeführt. Hierzu weist die Berechnungseinheit 27 einen nicht näher dargestellten Prozessor auf. Des Weiteren umfasst die Berechnungseinheit 27 auch entsprechende Berechnungsprogramme und/oder Berechnungssoftware. Die Berechnungsprogramme und/oder Berechnungssoftware können dabei innerhalb einer nicht näher dargestellten Speichereinheit der Berechnungseinheit 27 und/oder der Magnetresonanzvorrichtung 10 gespeichert sein. Zudem können die Berechnungsprogramme und/oder Berechnungssoftware auch in einem externen Speicher, wie beispielsweise einer Cloud, hinterlegt sein, wobei die Berechnungseinheit 27 hierbei mittels eines Datennetzwerks auf die Berechnungsprogramme und/oder Berechnungssoftware zugreifen kann.

Die Berechnungseinheit 27 kann innerhalb der Systemsteuereinheit 22 integriert sein. Alternativ oder zusätzlich kann die Berechnungseinheit 27 auch als selbstständige Einheit ausgebildet sein.

Die Erfassungseinheit 28 ist im vorliegenden Ausführungsbeispiel von einer Kamera, insbesondere einer 2D-Kamera und/oder einer 3D-Kamera, zur Erfassung von 2D-Positionsdaten und/oder 3D-Positionsdaten des Patienten 15 gebildet. Die Erfassungseinheit 28 ist innerhalb eines Untersuchungsraums, innerhalb dessen auch die Scannereinheit 11 angeordnet und/oder installiert ist, angeordnet. Die Erfassungseinheit 28 ist dabei derart innerhalb des Untersuchungsraums angeordnet, dass ein Erfassungsbereich 30 der Erfassungseinheit 28, insbesondere der Kamera, einen Bereich, innerhalb dessen die Vorbereitung des Patienten 15 für die Magnetresonanzuntersuchung stattfindet, abdeckt. Dieser Erfassungsbereich 30 und/oder dieser Bereich befindet sich vorzugsweise vor der Scannereinheit 11 und dabei direkt angrenzend an eine Frontseite 31 der Scannereinheit 11. Der Erfassungsbereich 28 umfasst dabei auch einen Ort, in dem sich die Patientenlagerungsvorrichtung 16 für die Vorbereitung des Patienten 15 befindet.

Die Erfassungseinheit 28 kann dabei an der Scannereinheit 11, insbesondere an der Frontseite 31 der Scannereinheit 11, angeordnet sein. Besonders vorteilhaft ist die Erfassungseinheit 28 an einer Wand und/oder einer Raumdecke 32 des Untersuchungsraums angeordnet, so dass stets ein unverstellter Blick auf den Patienten 15 und/oder die Patientenlagerungsvorrichtung 16 während der Vorbereitung des Patienten 15 für die Magnetresonanzuntersuchung gewährleistet ist.

Die Projektionseinheit 29 ist ebenfalls innerhalb des Untersuchungsraums, innerhalb dessen auch die Scannereinheit 11 angeordnet und/oder installiert ist, angeordnet. Die Projektionseinheit 29 ist dabei derart innerhalb des Untersuchungsraums angeordnet, dass ein Projektionsbereich 33 der Projektionseinheit 29 einen Bereich, innerhalb dessen die Vorbereitung des Patienten 15 für die Magnetresonanzuntersuchung stattfindet, abdeckt. Dieser Projektionsbereich 33 und/oder dieser Bereich befindet sich vorzugsweise vor der Scannereinheit 11 und dabei direkt angrenzend an die Frontseite 31 der Scannereinheit 11. Der Projektionsbereich 33 umfasst dabei auch einen Ort, in dem sich die Patientenlagerungsvorrichtung 16 für die Vorbereitung des Patienten 15 befindet.

Die Projektionseinheit 29 kann dabei an der Scannereinheit 11, insbesondere an der Frontseite 31 der Scannereinheit 11, angeordnet sein. Besonders vorteilhaft ist die Projektionseinheit 29 an der Wand und/oder der Raumdecke 32 des Untersuchungsraums angeordnet, so dass stets ein unverstellter Blick auf den Patienten 15 und/oder die Patientenlagerungsvorrichtung 16 während der Vorbereitung des Patienten 15 für die Magnetresonanzuntersuchung gewährleistet ist.

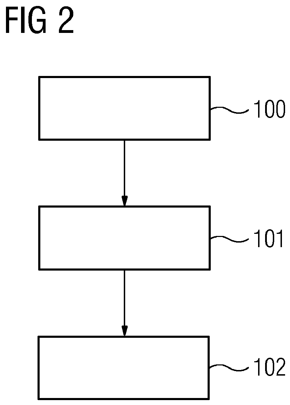

Das Verfahren zu einer Unterstützung einer Positionierung einer Zubehöreinheit 34 der Magnetresonanzvorrichtung 10 an einem Patienten 15 für eine Magnetresonanzuntersuchung ist in Fig. 2 dargestellt. Zu Beginn des Verfahrens befindet sich der Patient 15 bereits auf der Patientenlagerungsvorrichtung 16 positioniert. Die Zubehöreinheit 34 umfasst bevorzugterweise eine lokale Hochfrequenzantenneneinheit, die für die Magnetresonanzuntersuchung um den zu untersuchenden Bereich des Patienten 15 angeordnet wird. Alternativ oder zusätzlich kann die Zubehöreinheit 34 auch eine EKG-Einheit und/oder eine Infusionseinheit und/oder eine Positionierungseinheit, wie beispielsweise ein Kissen oder ein vorgeformter Schaum, und oder weitere Einheiten, die für die Magnetresonanzuntersuchung an dem Patienten 15 angeordnet werden sollen, umfassen.

In einem ersten Verfahrensschritt 100 erfolgt ein Erfassen von Positionsdaten des auf der Patientenlagerungsvorrichtung 16 positionierten Patienten 15 mittels der Erfassungseinheit 28. Die Positionsdaten umfassen hierbei eine Kontur des Patienten 15, insbesondere eine 2D-Kontur des Patienten 15 oder auch eine 3D-Kontur des Patienten 15.

In einem daran anschließenden, zweiten Verfahrensschritt 101 erfolgt ein Berechnen einer individuellen Sollposition der Zubehöreinheit 34 an und/oder auf dem Patienten 15 mittels der Berechnungseinheit 27. Das Berechnen der individuellen Sollposition der Zubehöreinheit 34 erfolgt hierbei anhand der Positionsdaten des Patienten 15. Des Weiteren können in die Berechnung der individuellen Sollposition der Zubehöreinheit 34 auch eine Untersuchungsinformation, wie beispielsweise ein Information einer Untersuchungsregion des Patienten 15, und/oder Daten von Zubehöreinheiten 34 eingehen. Die Daten der Zubehöreinheiten 34 können beispielsweise einen Positionsvorschlag der Zubehöreinheit 34 umfassen, wobei der Positionsvorschlag vorzugsweise eine vordefinierte Position angepasst an einen Durchschnittspatienten umfassen kann. Dieser Positionsvorschlag und/oder die vordefinierte Position angepasst an einen Durchschnittspatienten berücksichtigen lediglich den Körperbereich von Durchschnittspatienten, ohne jedoch auf die individuellen anatomischen Besonderheiten des einzelnen Patienten 15 einzugehen. Vorzugsweise sind die Daten der Zubehöreinheit 34 in einer Datenbank gespeichert, wobei die Berechnungseinheit 27 für die Berechnung der individuellen Sollposition der Zubehöreinheit 34 auf die Datenbank zugreifen kann.

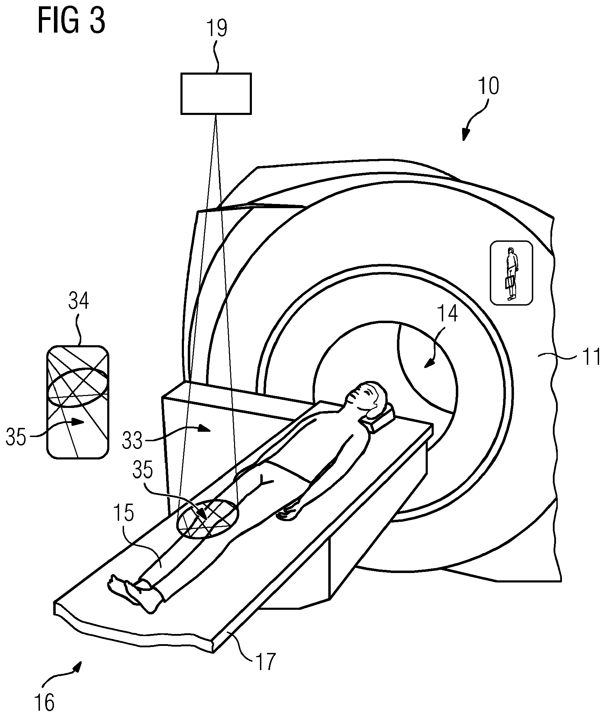

Die einzelnen Zubehöreinheiten 34 weisen jeweils eine Oberfläche mit einem charakteristischen Muster 35 auf, wobei mittels des charakteristischen Musters 35 eine Identifizierung der Zubehöreinheit 34 erfolgt (Fig. 3). Hierzu unterscheiden sich die charakteristischen Muster 35 der einzelnen Zubehöreinheiten 34 voneinander. Vorzugsweise sind in der Datenbank alle zur Verfügung stehenden Zubehöreinheiten 34 mit dem jeweiligen charakteristischen Muster 35 gespeichert. Zudem sind in der Datenbank auch die Daten aller zur Verfügung stehender Zubehöreinheiten 34 mit ihrem jeweiligen Positionsvorschlag und/oder mit der vordefinierten Position angepasst an einen Durchschnittspatienten gespeichert.

Für die Berechnung der individuellen Sollposition der Zubehöreinheit 34 weist die Berechnungseinheit 27 einen Algorithmus, insbesondere einen Berechnungsalgorithmus, auf, der anhand der Positionsdaten des Patienten 15 eine patientenspezifische individuelle Sollposition der Zubehöreinheit 34 bestimmt. Hierzu werden anhand der Positionsdaten, insbesondere den 2D-Positionsdaten und/oder den 3D-Positionsdaten, des Patienten 15 eine Kontur, insbesondere eine 2D-Kontur und/oder eine 3D-Kontur, und/oder ein Umriss, insbesondere ein 2D-Umriss und/oder ein 3D-Umriss, des Patienten 15 und/oder ein Patientenmodell, insbesondere ein 2D-Patientenmodell und/oder ein 3D-Patientenmodell, bestimmt. Anschließend erfolgt eine individuelle Anpassung des Positionsvorschlags und/oder der an einen Durchschnittspatienten angepasste vordefinierten Position der Zubehöreinheit 34, die innerhalb der Datenbank hinterlegt ist, an die Kontur, insbesondere die 2D-Kontur und/oder die 3D-Kontur, und/oder den Umriss, insbesondere den 2D-Umriss und/oder den 3D-Umriss, des Patienten 15 und/oder des Patientenmodells, insbesondere des 2D-Patientenmodells und/oder des 3D-Patientenmodells.

Die Auswahl der entsprechenden Zubehöreinheit 34, die für die anstehende Magnetresonanzuntersuchung am Patienten 15 erforderlich ist, kann dabei automatisch und/oder selbsttätig von der Berechnungseinheit 27 ausgewählt werden. Beispielsweise kann hierbei von der Berechnungseinheit 27 anhand der Positionsdaten des Patienten 15 und/oder anhand der Untersuchungsinformation ein zu untersuchender Körperbereich des Patienten 15 festgelegt und/oder bestimmt werden und von der Berechnungseinheit 27 anschließend eine für diesen Körperbereich geeignete Zubehöreinheit 34, insbesondere die geeignete lokale Hochfrequenzantenneneinheit, ausgewählt werden. Ist beispielsweise der Kopf des Patienten der zu untersuchende Bereich, so kann von der Berechnungseinheit 27 eine Kopf-Hochfrequenzantenneneinheit als geeignete Zubehöreinheit 34 ausgewählt werden.

Zudem kann es auch sein, dass die Zubehöreinheit 34 bereits von dem medizinischen Bedienpersonal 26 ausgewählt und/oder festgelegt wurde. Hierbei kann die Berechnungseinheit 27 die Zubehöreinheit 34 anhand des charakteristischen Musters 35 identifizieren, wobei hierzu die Zubehöreinheit 34 von der Erfassungseinheit 28 erfasst wird. Der in der Datenbank hinterlegte Positionsvorschlag und/oder die hinterlegt vordefinierte Position angepasst an einen Durchschnittspatienten der Zubehöreinheit 34 wird anschließend von der Berechnungseinheit 27 individuell an die Kontur, insbesondere die 2D-Kontur und/oder die 3D-Kontur, und/oder den Umriss, insbesondere den 2D-Umriss und/oder den 3D-Umriss, des Patienten 15 und/oder des Patientenmodells, insbesondere des 2D-Patientenmodells und/oder des 3D-Patientenmodells des Patienten 15 angepasst.

In einem dritten Verfahrensschritt 102 erfolgt ein Projizieren der individuellen Sollposition der Zubehöreinheit 34 auf dem Patienten 15, insbesondere auf einer Oberfläche des Patienten 15, mittels der Projektionseinheit 29, wobei die Zubehöreinheit 34 das charakteristisches Muster 35 umfasst, das an der individuellen Sollposition der Zubehöreinheit 34 projiziert wird. Eine Positionierung der Zubehöreinheit 34 erfolgt hierbei durch das medizinische Bedienpersonal 26, wobei das medizinische Bedienpersonal 26 solange die Zubehöreinheit 34 verschiebt oder neupositioniert, bis eine Übereinstimmung des charakteristischen Musters 35 auf der Oberfläche der Zubehöreinheit 35 mit dem auf dem Patienten 15 projizierten charakteristischen Muster 35 übereinstimmt.

Sofern die Auswahl der Zubehöreinheit 34 von der Berechnungseinheit 27 anhand der Positionsdaten des Patienten 15 und/oder anhand von Untersuchungsinformationen in dem zweiten Verfahrensschritt 101 erfolgt ist, kann in dem dritten Verfahrensschritt 102 somit dem medizinischen Bedienpersonal 26 zusätzlich angezeigt werden, welche Zubehöreinheit 34 für die anstehende Magnetresonanzuntersuchung verwendet werden soll. Für das medizinische Bedienpersonal 26 ist anschließend eine Bestimmung und/oder Auswahl der Zubehöreinheit 34 besonders einfach anhand des projizierten charakteristischen Musters 35 möglich.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zu einer Unterstützung einer Positionierung einer Zubehöreinheit an einem Patienten für eine Magnetresonanzuntersuchung, mit den folgenden Schritten:
- Erfassen von Positionsdaten des Patienten mittels einer Erfassungseinheit,
- Berechnen einer individuellen Sollposition der Zubehöreinheit mittels einer Berechnungseinheit, wobei das Berechnen der individuellen Sollposition der Zubehöreinheit anhand der Positionsdaten und anhand einer Untersuchungsinformation und/oder anhand von Daten von Zubehöreinheiten erfolgt, und
- Projizieren der individuellen Sollposition der Zubehöreinheit auf den Patienten mittels einer Projektionseinheit, wobei die Zubehöreinheit ein charakteristisches Muster umfasst, das an der individuellen Sollposition der Zubehöreinheit projiziert wird.

2. Verfahren nach Anspruch 1,
**dadurch gekennzeichnet, dass** die Erfassungseinheit eine Kamera umfasst.

3. Verfahren nach einem der vorhergehenden Ansprüche,
**dadurch gekennzeichnet, dass** die Zubehöreinheit eine lokale Hochfrequenzantenneneinheit umfasst.

4. Verfahren nach einem der vorhergehenden Ansprüche,
**dadurch gekennzeichnet, dass** die Positionsdaten des Patienten eine Kontur des Patienten umfassen.

5. Verfahren nach einem der vorhergehenden Ansprüche,
**dadurch gekennzeichnet, dass** die Berechnungseinheit für die Berechnung der individuellen Sollposition der Zubehöreinheit einen Algorithmus aufweist, der anhand der Positionsdaten des Patienten eine patientenspezifische individuelle Sollposition der Zubehöreinheit bestimmt.

6. Verfahren nach einem der vorhergehenden Ansprüche,
**dadurch gekennzeichnet, dass** sich die einzelnen Zubehöreinheiten anhand ihres charakteristischen Musters unterscheiden.

7. Verfahren nach einem der vorhergehenden Ansprüche,
**dadurch gekennzeichnet, dass** eine Bestimmung der Zubehöreinheit anhand des charakteristischen Musters erfolgt.

8. Verfahren nach einem der vorhergehenden Ansprüche,
**dadurch gekennzeichnet, dass** die Berechnungseinheit für eine Berechnung der individuellen Sollposition der Zubehöreinheit auf eine Datenbank zurückgreift, in der alle zur Verfügung stehenden Zubehöreinheiten mit charakteristischen Muster und/oder mit vordefinierter Position für einen Durchschnittspatienten gespeichert sind.

9. Verfahren nach einem der vorhergehenden Ansprüche,
**dadurch gekennzeichnet, dass** eine Positionierung der Zubehöreinheit bei einer Übereinstimmung des projizierten charakteristischen Musters mit dem charakteristischen Muster auf der Zubehöreinheit erfolgt.

10. Magnetresonanzvorrichtung, die zur Ausführung eines Verfahrens zu einer Unterstützung einer Positionierung einer Zubehöreinheit an einem Patienten für eine Magnetresonanzuntersuchung nach einem der Ansprüche 1 bis 9 ausgelegt ist, wobei die Magnetresonanzvorrichtung eine Scannereinheit, eine Patientenlagerungsvorrichtung zur Lagerung des Patienten für die Magnetresonanzuntersuchung, eine Erfassungseinheit zur Erfassung von Positionsdaten des Patienten, eine Berechnungseinheit zur Berechnung einer individuellen Sollposition einer Zubehöreinheit und eine Projektionseinheit zur Projektion der individuellen Sollposition der Zubehöreinheit aufweist.

11. Computerprogrammprodukt, welches ein Programm umfasst und direkt in einen Speicher einer programmierbaren Steuerungseinheit ladbar ist, mit Programmmitteln, um ein Verfahren zu einer Unterstützung einer Positionierung einer Zubehöreinheit an einem Patienten für eine Magnetresonanzuntersuchung nach einem der Ansprüche 1 bis 9 auszuführen, wenn das Programm in der Steuerungseinheit ausgeführt wird.

12. Elektronisch lesbarer Datenträger mit darauf gespeicherten elektronisch lesbaren Steuerinformationen, welche derart ausgestaltet sind, dass sie bei Verwendung des Datenträgers in einer Steuerungseinheit das Verfahren zu einer Unterstützung einer Positionierung einer Zubehöreinheit an einem Patienten für eine Magnetresonanzuntersuchung nach einem der Ansprüche 1 bis 9 durchführen.
